# EUROPÄISCHE PATENTANMELDUNG

(11) **EP 4 001 253 A1**
(43) Veröffentlichungstag der Anmeldung: **25.05.2022**
(21) Anmeldenummer: 20207181.7
(22) Anmeldetag: 12.11.2020
(51) Int. Cl.: C07C 67/38, C07C 69/44, C07F 9/58

(54) **DOPPELTE ALKOXYCARBONYLIERUNG VON DIENEN ALS EINTOPFSYNTHESE**

(71) Anmelder: Evonik Operations GmbH, 45128 Essen (DE)
(72) Erfinder: YANG, Ji, Harbin City (CN); JACKSTELL, Ralf, 18106 Rostock (DE); BELLER, Matthias, 18211 Ostseebad Nienhagen (DE); FRANKE, Robert, 45772 Marl (DE)
(74) Vertreter: Evonik Patent Association

(57) **Zusammenfassung**

Verfahren zur doppelten Alkoxycarbonylierung von Dienen als Eintopfsynthese.

## Beschreibung

Die Erfindung betrifft ein Verfahren zur doppelten Alkoxycarbonylierung von Dienen als Eintopfsynthese.

Die Alkoxycarbonylierung ethylenisch ungesättigter Verbindungen ist ein Prozess mit steigender Bedeutung. Unter einer Alkoxycarbonylierung versteht man die Umsetzung ethylenisch ungesättigter Verbindungen (Olefinen) mit Kohlenmonoxid und Alkoholen in Gegenwart eines Metall-Ligand-Komplexes zu den entsprechenden Estern. Üblicherweise wird als Metall Palladium eingesetzt. Das folgende Schema zeigt die allgemeine Reaktionsgleichung einer Alkoxycarbonylierung:

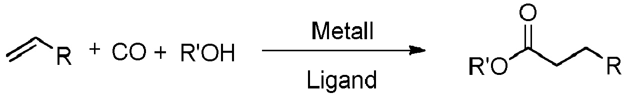

In WO 2006/084889 A1 wird ein Verfahren zur Herstellung einer Dicarbonsäure beschrieben. Dieses umfassend die Schritte:
(a) Inkontaktbringen eines konjugierten Diens mit Kohlenmonoxid und Wasser in Gegenwart eines Katalysatorsystems, einschließlich einer Palladiumquelle, einer Anionenquelle und eines zweizähnigen Phosphinliganden; um eine Mischung zu erhalten, die eine ethylenisch ungesättigte Säure und ein oder mehrere reversible Addukte des konjugierten Diens und der ethylenisch ungesättigten Säure enthält; und
(b) Entfernen von nicht umgesetztem konjugiertem Dien und den reversiblen Addukten des konjugierten Diens aus dem Reaktionsgemisch; und
(c) Umsetzen der in Schritt (b) erhaltenen Mischung, die die ethylenisch ungesättigte Säure enthält, weiter mit Kohlenmonoxid und Wasser, um die Dicarbonsäure zu erhalten.

Die technische Aufgabe der Erfindung ist die Bereitstellung eines neuen Verfahrens, welches anders als im oben genannten Stand der Technik das Entfernen von nicht umgesetztem konjugiertem Dien und / oder den reversiblen Addukten des konjugierten Diens aus dem Reaktionsgemisch nicht benötigt.

Die Aufgabe wird gelöst durch ein Verfahren nach Anspruch 1.

Verfahren umfassend die Verfahrensschritte:
a) Vorlegen eines Diens;
b) Zugabe eines Liganden gemäß Formel (I): 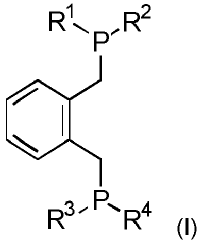 wobei
   R¹, R², R³, R⁴ ausgewählt sind aus: -(C₅-C₂₀)-Heteroarylrest, -(C₁-C₁₂)-Alkyl;
c) Zugabe einer Verbindung, welche Pd umfasst;
d1) Zugabe eines Alkohols,
   wobei der Alkohol mindestens in der doppelten Mengen gegenüber dem Dien, bezogen auf das molare Verhältnis, zugegeben wird;
e) Zuführen von CO;
f) Erwärmen des Reaktionsgemisches aus a) bis e), wobei das Dien direkt zu einem Diester umgesetzt wird, ohne dass mindestens eine der folgenden Komponenten aus dem Reaktionsgemisch entfernt wird:
   - nicht umgesetzte konjugierte Dien,
   - reversiblen Addukte des konjugierten Diens.

Hierbei umfasst d) den Verfahrensschritt d1) und gegebenenfalls den weiteren Verfahrensschritte d2).

Hierbei kann die Zugabe der Stoffe in beliebiger Reihenfolge erfolgen. Üblicherweise erfolgt die Zugabe von CO jedoch, nachdem die Reaktionspartner in den Schritten a) bis d) vorgelegt wurden. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Der Begriff (C₅-C₂₀)-Heteroaryl umfasst mono- oder polycyclische aromatische Kohlenwasserstoffreste mit 5 bis 20 Kohlenstoffatomen, wobei eins oder mehrere der Kohlenstoffatome durch Heteroatome ersetzt sind. Bevorzugte Heteroatome sind N, O und S. Die (C₅-C₂₀)-Heteroarylgruppen weisen 5 bis 20, bevorzugt 5 bis 6 Ringatome auf. Somit ist beispielsweise Pyridyl im Rahmen dieser Erfindung ein C₆-Heteroarylrest, Furyl ist ein C₅-Heteroarylrest.

In einer Variante des Verfahrens stehen mindestens drei der Reste R¹, R², R³, R⁴ für -(C₁-C₁₂)-Alkyl.

In einer Variante des Verfahrens stehen mindestens drei der Reste R¹, R², R³, R⁴ für -^{ter}Bu.

In einer Variante des Verfahrens stehen mindestens einer der Reste R¹, R², R³, R⁴ für -(C₅-C₂₀)-Heteroarylrest.

In einer Variante des Verfahrens stehen mindestens einer der Reste R¹, R², R³, R⁴ für 2-Pyridyl.

In einer Variante des Verfahrens ist der -(C₃-C₂₀)-Heteroarylrest 2-Pyridyl.

In einer Variante des Verfahrens weist der Ligand in Verfahrensschritt b) die Formel (1) auf:

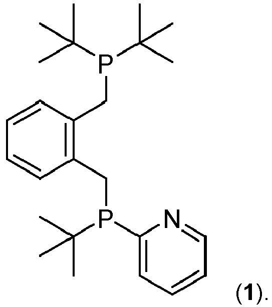

In einer Variante des Verfahrens ist das Dien im Verfahrensschritt a) ausgewählt ist aus: 1,3-Butadien, 1,2-Butadien, Vinylcyclohexen.

In einer Variante des Verfahrens die Verbindung in Verfahrensschritt c), welche Pd umfasst, ausgewählt ist aus: Palladium(II)-trifluoroacetate, Palladiumdichlorid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(II), Palladium(cinnamyl)dichlorid, Palladiumiodid, Palladiumdiiodid.

Vorzugsweise handelt es sich bei der Verbindung, welche Pd umfasst, um Pd(TFA)₂, Pd(dba)₂, Pd(acac)₂ oder Pd(OAc)₂. Besonders geeignet sind Pd(TFA)₂ und Pd(acac)₂.

Das molare Verhältnis Pd : Ligand liegt vorzugsweise im Bereich von 1:1 bis 1:10, bevorzugt von 1:1 bis 1:6, besonders bevorzugt von 1:1 bis 1:4.

In einer Variante des Verfahrens ist der Alkohol in Verfahrensschritt d1) ausgewählt ist aus: Methanol, Ethanol, 1-Propanol, 1-Butanol, 1-Pentanol, 1-Hexanol, 2-Propanol, *tert*-Butanol, 3-Pentanol.

In einer Variante des Verfahrens ist der Alkohol in Verfahrensschritt d1) Methanol.

In einer Variante des Verfahrens umfasst das Verfahren den zusätzlichen Verfahrensschritt d2):
d2) Zugabe einer Broenstedtsäure.

In einer Variante des Verfahrens handelt es sich bei der Broenstedtsäure aus Verfahrensschritt d2) um para-Toluolsulfonsäure.

In einer Variante des Verfahrens wird im Verfahrensschritt f) die Komponente:
- nicht umgesetzte konjugierte Dien,
nicht aus dem Reaktionsgemisch entfernt.

In einer Variante des Verfahrens wird im Verfahrensschritt f) die Komponente:
- reversiblen Addukte des konjugierten Diens,
nicht aus dem Reaktionsgemisch entfernt.

In einer Variante des Verfahrens werden im Verfahrensschritt f) die beiden folgenden Komponenten:
- nicht umgesetzte konjugierte Dien,
- reversiblen Addukte des konjugierten Diens,
nicht aus dem Reaktionsgemisch entfernt.

In einer Variante des Verfahrens wird dem Reaktionsgemisch ein organisches Lösungsmittel zugegeben, welches kein Alkohol ist. Vorzugsweise ist dieses ausgewählt aus: Toluol, Xylol, Anisol, Chlorbenzol, THF, Methylfuran, Propylencarbonat, Cyclohexan, Alkan, Ester, Ether. Besonders bevorzugt ist Toluol.

CO wird in Verfahrensschritt e) vorzugsweise bei einem CO-Partialdruck im Bereich von 0,1 bis 10 MPa (1 bis 100 bar), bevorzugt von 1 bis 5 MPa (10 bis 50 bar), besonders bevorzugt von 3 bis 5 MPa (30 bis 50 bar) zugeführt.

In einer Variante des Verfahrens wird die Reaktionsmischung in Verfahrensschritt f) des erfindungsgemäßen Verfahrens auf eine Temperatur im Bereich von 30 °C bis 150 °C, bevorzugt von 40 °C bis 140 °C, besonders bevorzugt von 60 °C bis 130 °C erwärmt, um die ethylenisch ungesättigte Verbindung zu einem Diester umzusetzen.

Im Folgenden soll die Erfindung anhand eines Ausführungsbeispieles näher erläutert werden.

### Experimentalvorschriften

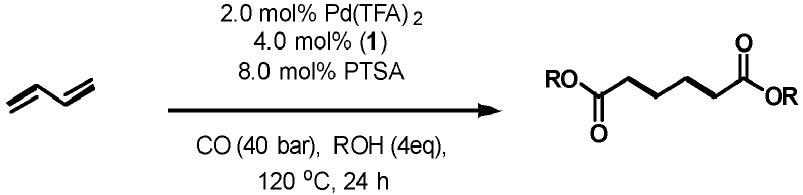

Im Allgemeinen wurde bei allen katalytischen Experimenten ein Überschuss an Ligand verwendet, um die Stabilität des aktiven Komplexes bei niedriger Metallkonzentration sicherzustellen. Alle Experimente verwenden 1,3-Butadien als Gas. Das Butadien wird bei niedriger Temperatur in ein spezielles Metallrohr (dieses Metallrohr kann mit dem entsprechenden Autoklaven verbunden werden) einkondensiert, und die entsprechende Butadienmasse wird genau gewogen, um die der dem Autoklaven zugesetzte Menge zu bestimmen. Es wurde ein 100 ml Autoklav verwendet. Zuerst wurde der Autoklav evakuiert und dann mit Argon gefüllt (dreimalige Wiederholung des Vorganges). Dann werden die Feststoffe unter Argonatmosphäre in den Autoklaven gegeben. Es werden vier Äquivalente Methanol (bezogen auf die Menge an Butadien) und Toluol verwendet. Das Volumenverhältnis von Methanol und Toluol wurde in der Versuchsreihe variiert. Die Reaktionen wurden 24 Stunden unter der angegebenen Temperatur gerührt. Danach wurde der Autoklav auf Raumtemperatur abgekühlt und der Druck vorsichtig abgelassen. Mesitylen (0,5 mmol) wurde als interner Standard in die Reaktion gegeben. Eine Probe der Mischung wurde durch Gaschromatographie analysiert. Reines Produkt wird durch Säulenchromatographie an Kieselgel erhalten (Elutionsmittel: Pentan / Ethylacetat = 40).

### Reaktionsbedingungen

Alkohol: MeOH / ⁿBuOH (4eq, bezogen auf das Olefin)
Druck (CO): 40 bar
Temperatur: 120 °C
Reaktionszeit: 24 h
Ligand: (1)
Pd : Ligand = 2 mol%: 4 mol% (bezogen auf das Olefin)

### Versuchsergebnisse

| Alkohol | Ausbeute Diester [%] |
|---|---|
| MeOH | 86 |
| ⁿBuOH | 85 |

## Patentansprüche

1. Verfahren umfassend die Verfahrensschritte:
a) Vorlegen eines Diens;
b) Zugabe eines Liganden gemäß Formel (I): 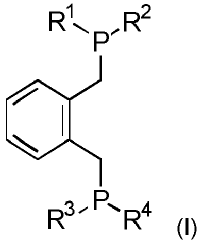 wobei
R¹, R², R³, R⁴ ausgewählt sind aus: -(C₅-C₂₀)-Heteroarylrest, -(C₁-C₁₂)-Alkyl;
c) Zugabe einer Verbindung, welche Pd umfasst;
d1) Zugabe eines Alkohols,
wobei der Alkohol mindestens in der doppelten Mengen gegenüber dem Dien, bezogen auf das molare Verhältnis, zugegeben wird;
e) Zuführen von CO;
f) Erwärmen des Reaktionsgemisches aus a) bis e), wobei das Dien direkt zu einem Diester umgesetzt wird, ohne dass mindestens eine der folgenden Komponenten aus dem Reaktionsgemisch entfernt wird:
- nicht umgesetzte konjugierte Dien,
- reversiblen Addukte des konjugierten Diens.

2. Verfahren nach Anspruch 1,
wobei mindestens drei der Reste R¹, R², R³, R⁴ für -(C₁-C₁₂)-Alkyl stehen.

3. Verfahren nach einem der Ansprüche 1 bis 2,
wobei mindestens drei der Reste R¹, R², R³, R⁴ für -^{ter}Bu stehen.

4. Verfahren nach einem der Ansprüche 1 bis 2,
wobei mindestens einer der Reste R¹, R², R³, R⁴ für -(C₅-C₂₀)-Heteroarylrest stehen.

5. Verfahren nach einem der Ansprüche 1 bis 4,
wobei mindestens einer der Reste R¹, R², R³, R⁴ für 2-Pyridyl stehen.

6. Verfahren nach einem der Ansprüche 1 bis 5,
wobei der -(C₃-C₂₀)-Heteroarylrest 2-Pyridyl ist.

7. Verfahren nach einem der Ansprüche 1 bis 6,
wobei der Ligand in Verfahrensschritt b) die Formel (1) aufweist:

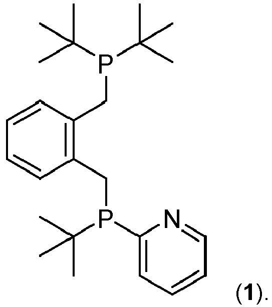

8. Verfahren nach einem der Ansprüche 1 bis 7,
wobei das Dien im Verfahrensschritt a) ausgewählt ist aus: 1,3-Butadien, 1,2-Butadien, Vinylcyclohexen.

9. Verfahren nach einem der Ansprüche 1 bis 8,
wobei die Verbindung in Verfahrensschritt c), welche Pd umfasst, ausgewählt ist aus: Palladium(II)-trifluoroacetate, Palladiumdichlorid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(II), Palladium(cinnamyl)dichlorid, Palladiumiodid, Palladiumdiiodid.

10. Verfahren nach einem der Ansprüche 1 bis 9,
wobei der Alkohol in Verfahrensschritt d1) ausgewählt ist aus: Methanol, Ethanol, 1-Propanol, 1-Butanol, 1-Pentanol, 1-Hexanol, 2-Propanol, tert-Butanol, 3-Pentanol.

11. Verfahren nach einem der Ansprüche 1 bis 10,
wobei das Verfahren den zusätzlichen Verfahrensschritt d2) umfasst:
d2) Zugabe einer Broenstedtsäure.

12. Verfahren nach einem der Ansprüche 1 bis 11, wobei im Verfahrensschritt f) die Komponente:
- nicht umgesetzte konjugierte Dien,
nicht aus dem Reaktionsgemisch entfernt wird.

13. Verfahren nach einem der Ansprüche 1 bis 12, wobei im Verfahrensschritt f) die Komponente:
- reversiblen Addukte des konjugierten Diens,
nicht aus dem Reaktionsgemisch entfernt wird.

14. Verfahren nach einem der Ansprüche 1 bis 13, wobei im Verfahrensschritt f) die beiden folgenden Komponenten:
- nicht umgesetzte konjugierte Dien,
- reversiblen Addukte des konjugierten Diens,
nicht aus dem Reaktionsgemisch entfernt werden.
